# EUROPEAN PATENT SPECIFICATION

(11) **EP 2 913 018 B1**
(45) Date of publication and mention of the grant of the patent: **25.01.2017**
(21) Application number: 15156288.1
(22) Date of filing: 24.02.2015
(51) Int. Cl.: A61B 18/14, A61B 17/3203

(54) **Surgical instrument**
Chirurgisches Instrument
Instrument chirurgical

(30) Priority: 26.02.2014 JP 2014035310
(43) Date of publication of application: 02.09.2015
(73) Proprietor: Seiko Epson Corporation, Shinjuku-ku, Tokyo 163-0811 (JP)
(72) Inventor: Kojima, Hideki, Nagano, 392-8502 (JP); Uchida, Kazuaki, Nagano, 392-8502 (JP); Sekino, Hirokazu, Nagano, 392-8502 (JP)
(74) Representative: Hoffmann Eitle

(56) References cited:
- DE-U1- 20 009 334
- JP-A- 2009 533 109
- US-A- 5 217 460
- US-A1- 2004 199 226
- US-A1- 2013 116 716

## Description

### BACKGROUND

### Technical Field

The present invention relates to an operation instrument.

### Related Art

US 2004/0199226 describes a working end of a medical instrument that applies energy to tissue. In one embodiment, the instrument has a microfluidic tissue engaging surface fabricated by soft lithography means together with optional superlattice cooling means that allows for very precise control of energy application, for example in neurosurgery applications. The tissue engaging surface can eject a high heat content vapour into the engaged tissue for treating tissue, while the superlative cooling structure can prevent collateral thermal damage. Also the superlattice cooling structure can be used to localize heat at a selected depth in tissue and prevent surface ablation. Also the superlattice cooling structure can be used to prevent tissue sticking to a thermal energy delivery surface. In another embodiment the tissue engaging surface can be used in a jaw structure for sealing tissue together with hydrojet means for transecting the tissue.

Known examples of an operation instrument include a liquid ejection surgical knife configured to perform incision, excision, and the like of living tissues by ejecting liquid (for example, water) continuously at a high speed. As an operation instrument configured mainly to stop bleeding, an electric surgical knife is also known. According to an operation instrument disclosed in JP-T-2009-533109 , a structure having both a liquid ejection surgical knife and an electric surgical knife of a bipolar type in one unit is described. With this structure, a trouble of changing the operation instrument to another instrument during an operation can be eliminated.

The operation instrument disclosed in JP-T-2009-533109 has a forceps shape, and performs a treatment by gripping the living tissue. However, when gripping the living tissue and passing electricity, the cauterized living tissue may be adhered to a distal end of forceps. The forceps accommodate a liquid ejection tube for the liquid ejection surgical knife together with electrodes for an electric surgical knife. However, due to the adhesion of the cauterized living tissue, an opening of the liquid ejection tube may be clogged.

In addition, in the operation instrument of the related art, a reduction in size, a reduction in cost, resource saving, ease of manufacture, improvement of user-friendliness are desired.

### SUMMARY

An advantage of some aspects of the invention is to solve at least a part of the problems described above, and the invention, which is defined in the appended claims, can be implemented as the following aspects.

An aspect of the invention provides an operation instrument. The operation instrument includes: an electric surgical knife, a fluid ejection surgical knife, and a gripper mechanism including a pair of jaws (gripper strips) configured to be openable and closable so as to grip a living tissue and at least one of which is a movable portion. The respective jaws (gripper strips) are provided with electrodes for the electric surgical knife respectively, one of the pair of jaws (gripper strips) is provided with an ejection opening of the fluid ejection surgical knife formed thereon, the ejection opening is formed at a position retracted from an end portion on the distal end side of the electrode. According to the operation instrument of the mode described above, in a case where the electric surgical knife is driven to perform a treatment, the living tissue cauterized by the electric surgical knife may be adhered to a distal end of the gripper mechanism. However, since the ejection opening of the fluid ejection surgical knife is formed at a position retracted from the end portion on the distal end side of the electrode, the ejection opening is prevented from being clogged by adhesion of the cauterized living tissue.

In the operation instrument of the aspect described above, the jaw (gripper strip) on the side where the ejection opening is provided may include a first insulating portion formed of an insulating material, and the ejection opening may be formed in the first insulating portion. According to the operation instrument of this configuration, since a portion of the jaw (gripper strip) on the side where the ejection opening is provided other than the electrode is formed of the insulating material, a weight reduction of the gripper mechanism as a processing portion is easily achieved.

In the operation instrument of the aspect described above, a suction opening communicating with a suction apparatus may be formed in the jaw (gripper strip) on the side where the ejection opening is not provided, and the suction opening may be formed at a position retracted from the end portion on the distal end side of the electrode. According to the operation instrument of this configuration, the suction opening is prevented from being clogged by adhesion of the cauterized living tissue as a result of usage of the electric surgical knife.

In the operation instrument of the aspect described above, the jaw (gripper strip) on the side where the suction opening is provided may include a second insulating portion formed of an insulating material, and the suction opening may be formed in the second insulating portion. According to the operation instrument of this configuration, since a part of the jaw (gripper strip) on the side where the suction opening is provided except for the electrode is formed of an insulating material, a weight reduction of the gripper mechanism as the processing portion is easily achieved.

In the operation instrument of the mode described above, the fluid ejection surgical knife may include a pulse generating unit configured to generate pulses in fluid, and a communication path communicating between the pulse generating unit and the ejection opening. According to the operation instrument of this configuration, since the pulsed fluid ejection is enabled, an effective operation is enabled while reducing the amount of consumption of the fluid.

All of the plurality of components having various aspects of the invention described above are not necessarily essential. In order to solve part or entire part of problem described above or in order to achieve part or entire part of advantages described in this specification, part of the plurality of components may be modified, eliminated, replaced by other components, and part of limited contents may be eliminated as needed. In order to solve part or entire part of problem described above or in order to achieve part or entire part of advantages described in this specification, part or entire part of technical characteristics included in an aspect of the invention described above may be combined with part or entire part of the technical characteristic included in other aspects of the invention to form an independent aspect of the invention.

The invention may be implemented in various modes other than the apparatus. For example, the invention may be implemented in a mode such as a method of controlling the operation instrument.

### BRIEF DESCRIPTION OF DRAWINGS

The invention will be described with reference to the accompanying drawings, wherein like numbers reference like elements.
Fig. 1 is an explanatory drawing illustrating a configuration of an operation instrument according to an embodiment of the invention.
Fig. 2 is an explanatory drawing illustrating a state in which a living tissue is gripped with a processing portion.
Figs. 3A and 3B are explanatory drawings illustrating a configuration of an interior of the processing portion.

### DESCRIPTION OF EXEMPLARY EMBODIMENTS

Subsequently, modes of the invention will be described in the order to an embodiment and a modification.

### A. Embodiment

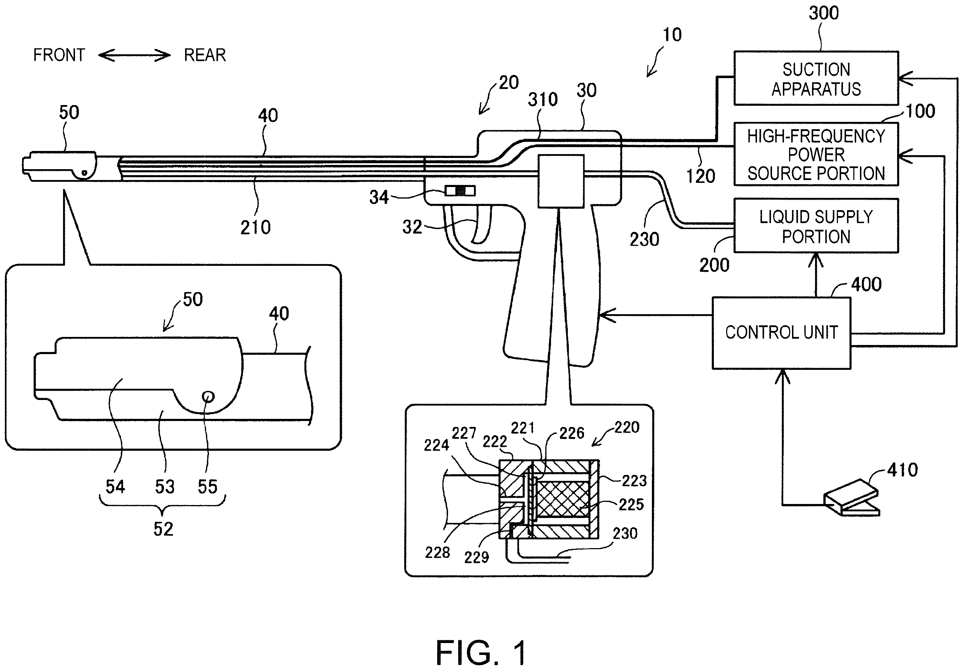

Fig. 1 is an explanatory drawing illustrating a configuration of an operation instrument 10 according to an embodiment of the invention. The operation instrument 10 of the embodiment is used in medical facilities, and has a function as an electric surgical knife, a function as a liquid ejection surgical knife, and a function as a sucking unit together. The operation instrument 10 includes a handpiece 20, a high-frequency power source portion 100, a liquid supply portion 200, a suction apparatus 300, and a control unit 400.

The handpiece 20 includes a body 30, an elongated shaft 40 extending from the body 30, and a processing portion 50 provided at a distal end of the shaft 40. In the following explanation, the processing portion 50 side in a direction of extension of the shaft 40 is defined as "front side", the body 30 side in the direction of extension of the shaft 40 is defined as "rear side". The front side and the rear side may also be referred to simply as "front" and "rear".

The processing portion 50 is configured to constitute part of the electric surgical knife of a bipolar type, which is configured to grip a living tissue to incise the living tissue at a high frequency, and coagulate the living tissue, and includes a gripper mechanism 52 configured to grip the living tissue. The gripper mechanism 52 includes a pair of jaws (gripper strips) 53 and 54. The first jaw (gripper strip) 53 on one side is configured as a fixed portion which does not move with respect to the shaft 40 and the second jaw (gripper strip) 54 on the other side is configured as a movable portion capable of pivoting about a gripper shaft 55. The gripper mechanism 52 is configured to perform an opening and closing operation by the second jaw (gripper strip) 54 pivoting within a predetermined angle range about the gripper shaft 55.

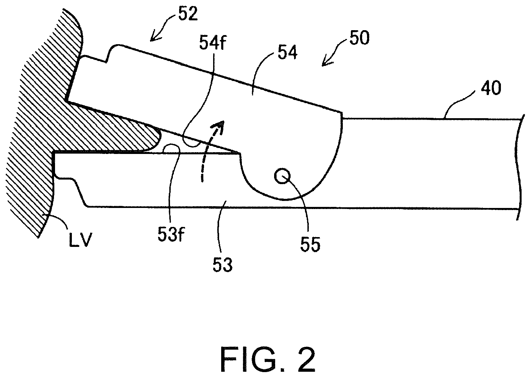

Fig. 2 is an explanatory drawing illustrating a state in which a living tissue LV is gripped with the processing portion 50. As illustrated by a broken arrow in the drawing, the gripper mechanism 52 is opened by the pivotal movement of the second jaw (gripper strip) 54 about the gripper shaft 55, and is capable of gripping the living tissue LV between a gripping surface 53f of the first jaw (gripper strip) 53 and a gripping surface 54f of the second jaw (gripper strip) 54.

Returning back to Fig. 1 , the pivotal movement of the second jaw (gripper strip) 54 described above is achieved by a fore-and-aft movement of a rod or a wire (not illustrated) inserted into the shaft 40, and coupled at one end thereof to the second jaw (gripper strip) 54. The body 30 of the handpiece 20 is provided with a trigger lever 32, and the trigger lever 32 is coupled to the other end of the rod or the wire. Accordingly, the opening and closing operation of the gripper mechanism 52 is enabled on the basis of the operation of the trigger lever 32. A configuration in which an actuator is provided to cause the gripper mechanism 52 to perform the opening and closing operation by the actuator when the trigger lever 32 is operated may be alternatively employed.

The body 30 is provided with a slide switch 34. The slide switch 34 has three positions corresponding to the electric surgical knife, the liquid ejection surgical knife, and the sucking unit. A user operates the slide switch 34 to select one of the tree positions, so that a function to be driven in the operation instrument 10 may be selected from the electric surgical knife, the liquid ejection surgical knife, and the sucking unit.

The control unit 400 is connected to the handpiece 20, the high-frequency power source portion 100, the liquid supply portion 200, and the suction apparatus 300 respectively. The control unit 400 controls actions of the respective portions 20 to 40. A foot switch 410 is connected to the control unit 400. Upon the ON/OFF of the foot switch 410 by the user, the function of one of the electric surgical knife, the liquid ejection surgical knife, and the sucking unit selected by the slide switch 34 is operated.

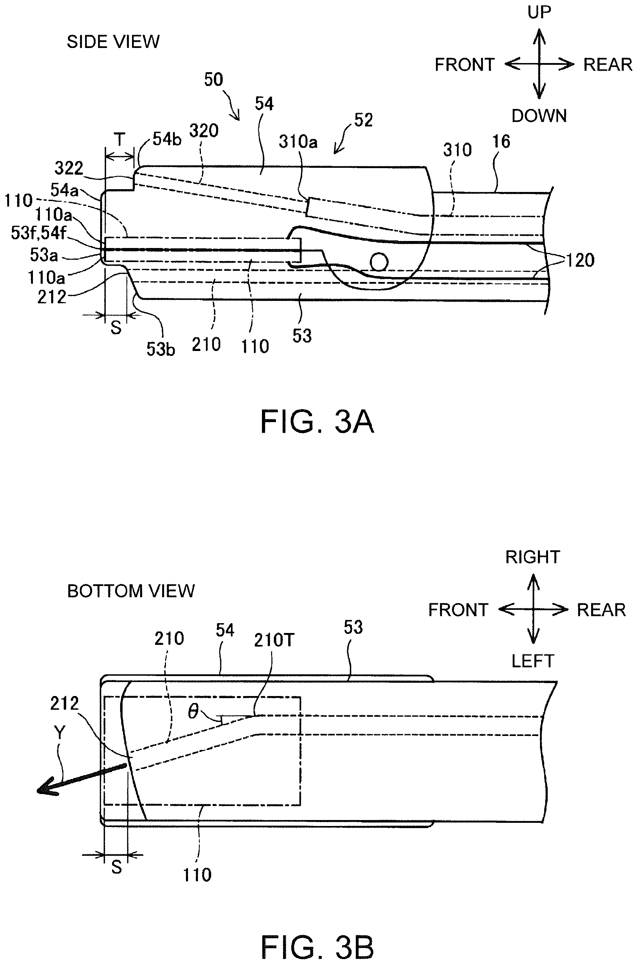

Figs. 3A and 3B are explanatory drawings illustrating a configuration of an interior of the processing portion 50. Fig. 3A is a side view and Fig. 3B is a bottom view. As illustrated, the first jaw (gripper strip) 53 and the second jaw (gripper strip) 54 each includes a pair of electrodes 110 and 110 formed of a conductive material (for example, a metal) integrated therein. The electrodes 110 and 110 are flat plate-shaped members, and are disposed on the respective jaws (gripper strips) 53 and 54 that primary surfaces on one side of the electrodes 110 and 110 constitute parts of the respective gripping surfaces 53f and 54f. In other words, the electrodes 110 and 110 are disposed on the respective jaws (gripper strips) 53 and 54 in a state in which the primary surfaces on one side are exposed from the respective gripping surfaces 53f and 54f sides. Outer shells of the jaws (gripper strips) 53 and 54 are formed of an insulating material, for example, resin material such as polyethylene, or polyamide, or ceramics.

The range occupied by the respective electrodes 110 and 110 of the jaws (gripper strips) 53 and 54 is deviated to the front side (the distal end side). Specifically, the respective jaws (gripper strips) 53 and 54 have a stepped structure having an outside in the vertical direction (the direction perpendicular to the fore-and-aft direction) retracted rearward and an inside (the side of the gripping surfaces 53f and 54f) projecting toward the distal end side. The electrodes 110 and 110 are disposed respectively in a form in which end portions 110a and 110a at the distal end side of the electrodes 110 and 110 are inserted into projecting distal end portions 53a and 54a.

The electrodes 110 and 110 are electrically connected to the high-frequency power source portion 100 (Fig. 1) provided outside the handpiece 20 by electric cables 120 and 120. The high-frequency power source portion 100 supplies high-frequency current to the electrodes 110 via the electric cables 120. The high-frequency power source portion 100 includes a switching element configured to receive a control signal from the control unit 400 and turn the high-frequency current to the electrodes 110 ON and OFF. The high-frequency power source portion 100 performs other various types of control required for the operation of the electric surgical knife. The high-frequency current supplied to the electrodes 110 flows to an affected area. At this time, a Joule heat is generated by a load or a contact resistance, which solidifies protein at the affected area to enable stop of bleeding.

The handpiece 20 illustrated in Fig. 1 accommodates the liquid ejection surgical knife. The liquid ejection surgical knife includes a liquid ejection tube 210, an actuator 220, and a liquid supply flow channel 230. The liquid supply flow channel 230 is a flow channel for supplying liquid pumped from the liquid supply portion 200 provided outside the handpiece 20 to the actuator 220. The liquid supply flow channel 230 is formed of a flexible member. In this example, the liquid supply flow channel 230 is formed of PEEK member. The liquid supply flow channel 230 may be replaced by various types of flexible member such as polyvinyl chloride, silicon, or thermoplastic elastomer. As the liquid to be supplied to the actuator 220, various types of liquids such as aseptic water for medical use and physiological saline may be used.

The actuator 220 provides the liquid supplied from the liquid supply flow channel 230 with pulsation. The liquid provided with the pulsation is supplied to the liquid ejection tube 210, and is ejected as pulsed liquid from an ejection opening 212 ( Figs. 3A and 3B) formed at a distal end of the liquid ejection tube 210. A predetermined range of the liquid ejection tube 210 on the distal end side (hereinafter, referred to as "distal end range") is accommodated in the first jaw (gripper strip) 53 of the gripper mechanism 52. However, the position of accommodation will be described later. In the embodiment disclosed here, the liquid ejection tube 210 is formed of stainless steel. However, the liquid ejection tube 210 may be formed of other materials having at least a predetermined rigidity such as other types of metals such as brass or reinforced plastic. The term "pulsed liquid" means liquid in a state in which the flow rate or the flow velocity varies. A mode of ejecting the liquid in a pulsed manner includes an intermittent ejection that ejects the liquid by repeating ejection and stop. However, what is essential is that the flow rate or the flow velocity of liquid are varied, so that the intermittent ejection is not necessarily required.

As illustrated, the actuator 220 includes a first case 221, a second case 222, a third case 223, a piezoelectric element 225, a reinforcing plate 226, and a diaphragm 227. The first case 221 is a cylindrical member. One end of the first case 221 is joined with the second case 222. The other end of the first case 221 is sealed by the third case 223. The piezoelectric element 225 is disposed in a space defined in the interior of the first case 221.

The piezoelectric element 225 is a multi-layer piezoelectric element. One of end portions of the piezoelectric element 225 is fixed to the diaphragm 227 via the reinforcing plate 226. The other end portion of the piezoelectric element 225 is fixed to the third case 223. The diaphragm 227 is formed of a metallic thin film, and is secured to the first case 221 at a peripheral edge portion thereof. A storage chamber 228 is formed between the diaphragm 227 and the second case 222. A volume of the storage chamber 228 is varied by driving the piezoelectric element 225.

A first flow channel 229 configured to allow the liquid to flow into the storage chamber 228 is formed in the second case 222. The first flow channel 229 is coupled to the liquid supply flow channel 230. A second flow channel 224 configured to allow the liquid accommodated in the storage chamber 228 to flow out is formed in the second case 222. The second flow channel 224 is connected to the liquid ejection tube 210.

A drive signal having a predetermined frequency is applied to the piezoelectric element 225 from the control unit 400. The piezoelectric element 225 vibrates at a predetermined frequency upon the reception of the drive signal from the control unit 400. When the piezoelectric element 225 vibrates, the volume of the storage chamber 228 is changed via the diaphragm 227, and the liquid accommodated in the storage chamber 228 is pressurized. The liquid pressurized or depressurized at the predetermined frequency is provided with pulsation. The liquid passes through the second flow channel 224 and the liquid ejection tube 210 and is ejected toward the outside as a pulsed liquid.

As illustrated in Figs. 3A and 3B , the first jaw (gripper strip) 53 configured as a fixed portion in the gripper mechanism 52 includes a distal end range of the liquid ejection tube 210 integrated therein. The liquid ejection tube 210 is a metallic tube, and is arranged so that a longitudinal direction is oriented in the fore-and-aft direction. Specifically, the distal end range of the liquid ejection tube 210 is provided in a retracted portion 53b on the front side of the first jaw (gripper strip) 53, and the ejection opening 212 of the liquid ejection tube 210 is positioned on the front side (distal end side) of the retracted portion 53b. Therefore, the ejection opening 212 is arranged at a position retracted from the end portion 110a on the distal end side of the electrode 110 by a predetermined distance S, which corresponds to part of the electric surgical knife. In this embodiment, the predetermined distance S is 1.5 mm. The predetermined distance S may be set to fall within a range from 0.5 to 10.0 mm and, preferably, from 1.0 to 2.0 mm.

In this embodiment, a ridge line outside the retracted portion 53b has a shape retracted on the left side with respect to a ridge line of the distal end portion 53a of the first jaw (gripper strip) 53 as illustrated in Fig. 3B . On this basis, the liquid ejection tube 210 has a shape bent at a mid portion 210T in the distal end range leftward by a predetermined angle θ. With this configuration, the direction of the ejection opening 212, that is, a direction of ejection Y of liquid may be increased to a wide range by rotating the handpiece 20 about an axial direction of the liquid ejection tube or the like. The left and right directions are directions perpendicular to both the fore-and-aft direction and the vertical direction described above.

An adjunct to the description of the predetermined angle θ of the ejection tube will be given. For example, in a case where the surgical knife is inserted into a hole provided partly on a surface of the body to perform an operation laparoscopically, the angle θ of the liquid ejection tube is 0, that is, parallel to the axis, the angle of the handpiece is changed significantly in order to change the direction of ejection, which is difficult to be performed laparoscopically having a limited operation space. In contrast, when the angle θ is provided like the configuration described above, the direction of ejection can be changed by rotating the handpiece at that time, so that improvement in usability is achieved.

The liquid ejection surgical knife configured in a manner described above is allowed to inject liquid and perform incision or excision of the affected area with a liquid flow. In contrast, the electric surgical knife is capable of performing incision or clotting (stopping blood) of the affected area by a thermal action of the high-frequency current.

As illustrated in Fig. 1, the handpiece 20 of the embodiment accommodates a suction flow channel 310 for configuring the sucking unit. The suction flow channel 310 is connected to the suction apparatus 300 prepared outside the handpiece 20. As illustrated in Fig. 3A , a suction tube 320 is connected to a distal end 310a of the suction flow channel 310 on the other end side (front side). The suction tube 320 is accommodated in the second jaw (gripper strip) 54 and extends to a retracted portion 54b of the second jaw (gripper strip) 54. A suction opening 322 opening at a distal end of the suction tube 320 is located at a front side (distal end side) of the retracted portion 54b. Consequently, the suction opening 322 is located at a position retracted from the end portion 110a on the distal end side of the electrode 110 as part of the electric surgical knife by a predetermined distance T. In this embodiment, the predetermined distance T is 1.0 mm. The predetermined distance T may be set to fall within a range from 0.5 to 5.0 mm and, preferably, from 1.0 to 2.0 mm. Although not illustrated, the suction tube 320 has a shape bent at the mid portion at the distal end range in the same manner as the liquid ejection tube 210, and has a structure which allows to increase the sucking range to a wide range.

In this embodiment, the suction tube 320 is formed of stainless steel. However, the suction tube 320 may be formed of other materials having at least a predetermined rigidity such as other types of metals such as brass or reinforced plastic. In contrast, the suction flow channel 310 is formed of a flexible member. In the embodiment, the suction flow channel 310 is formed of PEEK member. The suction flow channel 310 may be replaced by various types of flexible member such as polyvinyl chloride, silicon, or thermoplastic elastomer. The distal end 310a on the front side of the suction flow channel 310 is located on the front side of a movable area in the vicinity of a root of the second jaw (gripper strip) 54, and the suction flow channel 310 formed of a member having flexibility is located at the movable range.

With the configuration described above, liquid or an excited portion in the vicinity of the suction opening 322 is sucked by the suction apparatus 300 from the suction opening 322 via the suction flow channel 310.

According to the operation instrument 10 of the embodiment configured in the manner described thus far, in a case where the electric surgical knife is driven to perform a treatment, the living tissue cauterized by the electric surgical knife may be adhered to distal ends of the first and second jaws (gripper strips) 53 and 54. However, since the ejection opening 212 of the liquid ejection surgical knife is formed at a position retracted from the end portion 110a on the distal end side of the electrode 110, the ejection opening 212 is prevented from being clogged by adhesion of the cauterized living tissue described above. In addition, since the suction opening 322 is formed at a position retracted from the end portion 110a at the distal end side of the electrode 110, the suction opening 322 is prevented from being clogged by adhesion of the cauterized living tissue. Therefore, according to the operation instrument 10 of this embodiment, lowering of performance of the liquid ejection surgical knife and the sucking unit is prevented.

According to the operation instrument 10 of the embodiment, since the outer shells of the first and second jaws (gripper strips) 53 and 54 are formed of an insulating material, and the ejection opening 212 is formed in the first jaw (gripper strip) 53 and the suction opening 322 is formed in the second jaw (gripper strip) 54, respectively, a weight reduction of the gripper mechanism 52 as the processing portion is easily achieved.

### B. Modifications

The invention is not limited to the embodiments described above, and various modes may be implemented without departing the scope of the invention. For example, the following modifications may be applied.

### Modification 1

In the embodiment described above, liquid is used as fluid to be ejected from the operation instrument 10. In contrast, in this modification, gas may be used as the fluid to be ejected from the operation instrument 10. It is also possible to eject the fluid in a state of mixture of liquid and gas.

### Modification 2

In the embodiment described above, the liquid ejection tube 210 is accommodated in the first jaw (gripper strip) 53 as the fixed portion, and the suction tube 320 is accommodated in the second jaw (gripper strip) 54 as the movable portion. In contrast, in the modification, a configuration in which the suction tube 320 is accommodated in the first jaw (gripper strip) 53 as the fixed portion and the liquid ejection tube 210 is accommodated in the second jaw (gripper strip) 54 as the movable portion is also applicable.

### Modification 3

In the embodiment, with the configuration in which the first jaw (gripper strip) 53 is configured as the fixed portion and the second jaw (gripper strip) 54 is configured as the movable portion, the gripper mechanism 52 is configured to perform an opening and closing operation. In contrast, in the modification, since the first jaw (gripper strip) 53 is also configured as the movable portion so that both of the jaws (gripper strips) 53 and 54 are pivoted, the gripper mechanism 52 is allowed to perform an opening and closing operation. A configuration in which the second jaw (gripper strip) 54 is configured as the movable portion which moves toward and away from the first jaw (gripper strip) 53 while maintaining the parallelism with respect to the first jaw (gripper strip) 53 configured as the fixed portion is applicable. Alternatively, a configuration in which first jaw (gripper strip) 53 is configured as the movable portion which moves toward and away from the second jaw (gripper strip) 54 while maintaining the parallelism with respect to the second jaw (gripper strip) 54 configured as the fixed portion is applicable. In addition, the both jaws (gripper strips) 53 and 54 may be configured to be movable portions which move toward and away from each other while maintaining the parallelism with respect to each other.

### Modification 4

In the embodiment described above, the operation instrument 10 is configured to have three functions of the electric surgical knife, the liquid ejection surgical knife, and the sucking unit. In contrast, in the modification, a configuration having two functions of the electric surgical knife and the liquid ejection surgical knife is applicable. In other words, in the first embodiment, a configuration in which the suction apparatus 300, the suction flow channel 310, and the suction tube 320 are eliminated is also applicable. In addition, although departing from the invention described above, the configuration having two functions of the electric surgical knife and the sucking unit, that is, a configuration in which the liquid supply portion 200, the liquid ejection tube 210, the actuator 220, and the liquid supply flow channel 230 are eliminated in the first embodiment is also applicable.

### Modification 5

In the embodiment described above, the slide switch 34 has three positions corresponding to the electric surgical knife, the liquid ejection surgical knife, and the sucking unit. In contrast, in the modification, as the usage as both the liquid ejection surgical knife and the sucking unit, a configuration including two positions corresponding to the usage as the electric surgical knife or the usage both as the electric surgical knife and the sucking unit may be employed. A configuration including two positions corresponding only to switching between the electric surgical knife and the liquid ejection surgical knife is also applicable. In this case, the sucking unit is not relevant to a foot switch, and may always be in the ON state during the treatment.

### Modification 6

In the embodiment described above, as a pulse generating unit of the fluid ejection surgical knife, a mechanism for generating a pulsation in the liquid in the storage chamber 228 by the piezoelectric element 225 is employed. In contrast, a configuration in which air bubbles are generated by irradiating a laser to the liquid, and pulsation is generated in the liquid in the storage chamber by the air bubbles may also be employed. In this case, an optical fiber cable for irradiating the laser in the storage chamber may be connected. The fluid ejection surgical knife may have a mechanism configured to heat the liquid in the storage chamber with an electric heater to generate the air bubbles and providing the fluid with pulsation thereby.

The invention is not limited to the embodiments, examples, and modifications described above, and may be implemented in various configurations without departing the scope of the invention. For example, technical characteristics in the embodiments, the examples, and the modifications corresponding to the technical characteristics in the respective embodiments in the respective modes described in the paragraph of the summary may be replaced or combined as needed in order to solve part or entire problem described above or in order to achieve part or entire part of the above-described advantages. The technical characteristics may be eliminated unless otherwise specified to be essential.

## Claims

1. An operation instrument (10) comprising:
a fluid ejection surgical knife and an electric surgical knife comprising;
a gripper mechanism (52) including a pair of jaws (53, 54) configured to be openable and closable so as to grip a living tissue and at least one of which is a movable portion,
wherein
the jaws (53, 54) each are provided with a gripping surface (53f, 54f) each having an electrode (110) for the electric surgical knife, and **characterized in that**
one of the pair of jaws (53) has a retracted portion (53b) provided at a distal end of the jaw (53) and an ejection opening (212) of the fluid ejection surgical knife is provided at a distal end face of the retracted portion (53b), such that the ejection opening (212) is not provided on said gripping surface (53f, 54f).

2. The operation instrument (10) according to Claim 1, wherein
the jaw (53) on a side where the ejection opening (212) is provided includes a first insulating portion formed of an insulating material, and
the ejection opening (212) is formed in the first insulating portion.

3. The operation instrument (10) according to claim 1 or claim 2, wherein the ejection opening (212) is provided at a distal end of the liquid ejection tube (210), the distal end of the liquid ejection tube (210) being bent at a predetermined angle (θ) with respect to the longitudinal direction of the jaw (53) such that the direction of ejection (Y) of liquid is at a predetermined angle (θ) with respect to the longitudinal direction of the jaw (53).

4. The operation instrument (10) according to any of claims 1-3, wherein
a suction opening (322) communicating with a suction apparatus (300) is formed in the jaw (54) in which the ejection opening (212) is not provided, and the suction opening (322) may be formed at a position (54b) retracted from the end portion (110a) on the distal end side of the electrode (110).

5. The operation instrument (10) according to claim 4, wherein
the jaw (54) on the side where the suction opening (322) is provided includes a second insulating portion formed of an insulating material, and
the suction opening (322) is formed in the second insulating portion.

6. The operation instrument (10) according to any one of the preceding claims, wherein
the fluid ejection surgical knife includes:
a pulse generating unit (200) configured to generate pulsation in fluid; and
a communication channel (210) communicating between the pulse generating unit (220) and the ejection opening (212).

## Patentansprüche

1. Eine Operationsvorrichtung (10), umfassend:
ein fluidausstoßendes chirurgisches Messer und ein elektrisches chirurgisches Messer, aufweisend:
einen Greifermechanismus (52) mit einem Paar von Klemmbacken (53, 54), die so ausgelegt sind, dass sie offenbar und verschließbar sind, um ein lebendes Gewebe zu greifen und mindestens eines davon ein beweglicher Abschnitt ist, wobei
die Klemmbacken (53, 54) jeweils mit einer Greiffläche (53f, 54f) versehen sind, die jeweils eine Elektrode (110) für das elektrische chirurgische Messer aufweisen,
**dadurch gekennzeichnet, dass**
eine der beiden Klemmbacken (53) einen zurückgezogenen Abschnitt (53b) aufwesit, der am distalen Ende der Klemmbacke (53) bereitgestellt ist, und eine Ausstoßöffnung (212) des fluidausstoßenden chirurgischen Messers an einer distalen Endseite des zurückgezogenen Abschnitts (53b) bereitgestellt ist, so dass die Ausstoßöffnung (212) nicht auf der genannten Greiffläche (53f, 54f) vorgesehen ist.

2. Eine Operationsvorrichtung (10) nach dem Anspruch 1, wobei
die Klemmbacke (53) an einer Seite, an der die Ausstoßöffnung (212) vorgesehen ist, einen ersten Isolierabschnitt aufweist, der aus einem Isoliermaterial gebildet ist, und
die Ausstoßöffnung (212) in dem ersten Isolierabschnitt gebildet ist.

3. Die Operationsvorrichtung (10) nach Anspruch 1 oder Anspruch 2, wobei die Ausstoßöffnung (212) an einem distalen Ende des Flüssigkeitsausstoßrohrs (210) vorgesehen ist, das distale Ende des Flüssigkeitsausstoßrohrs (210) in einem vorgegebenen Winkel (θ) in Bezug auf die Längsrichtung der Klemmbacke (53) biegbar ist, so dass die Ausstoßrichtung (Y) der Flüssigkeit in einem vorgegebenen Winkel (θ) in Bezug auf die Längsrichtung der Klemmbacke (53).

4. Die Operationsvorrichtung (10) nach einem der Ansprüche 1-3, wobei
eine Saugöffnung (322), die mit einer Saugvorrichtung (300) in Verbindung steht, in der Klemmbacke (54) ausgebildet ist, in der die Ausstoßöffnung (212) nicht vorgesehen ist, und die Saugöffnung (322) an einer Position (54b) ausgebildet sein kann, die von dem Endabschnitt (110a) an der distalen Endseite der Elektrode (110) zurückgezogen ist.

5. Die Operationsvorrichtung (10) nach Anspruch 4, wobei
die Klemmbacke (54) an der Seite, an der die Saugöffnung (322) vorgesehen ist, einen zweiten Isolierabschnitt aufweist, der aus einem Isoliermaterial ausgelegt ist, und die Saugöffnung (322) in dem zweiten Isolierabschnitt ausgelegt ist.

6. Die Operationsvorrichtung (10) nach einem der vorangegangenen Ansprüche, wobei
das fluidausstoßende chirurgische Messer umfasst:
eine Impulserzeugungseinheit (200), die konfiguriert ist, um eine Pulsation in einem Fluid zu erzeugen; und
einen Kommunikationskanal (210), der zwischen der Impulserzeugungseinheit (200) und der Ausstoßöffnung (212) kommuniziert.

## Revendications

1. Instrument de chirurgie (10) comprenant :
un couteau chirurgical à éjection de fluide et un couteau chirurgical électrique comprenant :
un mécanisme à dispositif de préhension (52) incluant un couple de mâchoires (53, 54) configurées pour pouvoir être ouvertes et fermées afin de saisir un tissu vivant, et dont au moins une est une partie mobile,
dans lequel
les mâchoires (53, 54) sont munies chacune d'une surface de préhension (53f, 54f) ayant chacune une électrode (110) pour le couteau chirurgical électrique, et **caractérisé en ce que**
l'une du couple de mâchoires (53) a une partie rétractée (53b) prévue au niveau d'une extrémité distale de la mâchoire (53), et une ouverture d'éjection (212) du couteau chirurgical à éjection de fluide est prévue au niveau d'une face d'extrémité distale de la partie rétractée (53b), de sorte que l'ouverture d'éjection (212) n'est pas prévue sur ladite surface de préhension (53f, 54f).

2. Instrument de chirurgie (10) selon la revendication 1, dans lequel
la mâchoire (53) sur un côté où l'ouverture d'éjection (212) est prévue inclut une première partie isolante formée d'une matière isolante, et
l'ouverture d'éjection (212) est formée dans la première partie isolante.

3. Instrument de chirurgie (10) selon la revendication 1 ou la revendication 2, dans lequel l'ouverture d'éjection (212) est prévue au niveau d'une extrémité distale du tube d'éjection de liquide (210), l'extrémité distale du tube d'éjection de liquide (210) étant incurvée à un angle prédéterminé (θ) par rapport à la direction longitudinale de la mâchoire (53) de sorte que la direction d'éjection (Y) de liquide est à un angle prédéterminé (θ) par rapport à la direction longitudinale de la mâchoire (53).

4. Instrument de chirurgie (10) selon l'une quelconque des revendications 1 à 3, dans lequel
une ouverture d'aspiration (322) communiquant avec un appareil d'aspiration (300) est formée dans la mâchoire (54) dans laquelle l'ouverture d'éjection (212) n'est pas prévue, et l'ouverture d'aspiration (322) peut être formée à une position (54b) rétractée de la partie d'extrémité (110a) sur le côté d'extrémité distale de l'électrode (110).

5. Instrument de chirurgie (10) selon la revendication 4, dans lequel
la mâchoire (54) sur le côté où l'ouverture d'aspiration (322) est prévue inclut une seconde partie isolante formée d'une matière isolante, et
l'ouverture d'aspiration (322) est formée dans la seconde partie isolante.

6. Instrument de chirurgie (10) selon l'une quelconque des revendications précédentes, dans lequel
le couteau chirurgical à éjection de fluide inclut :
une unité de production d'impulsion (200) configurée pour produire une impulsion dans un fluide ; et
un canal de communication (210) communiquant entre l'unité de production d'impulsion (220) et l'ouverture d'éjection (212).
